# EUROPEAN PATENT APPLICATION

(11) **EP 3 760 124 A1**
(43) Date of publication of application: **06.01.2021**
(21) Application number: 19184377.0
(22) Date of filing: 04.07.2019
(51) Int. Cl.: A61B 6/00, A61B 6/03

(54) **X-RAY IMAGING WITH AN INCREASED ANGULATION ROTATION RANGE**

(71) Applicant: Koninklijke Philips N.V., 5656 AG Eindhoven (NL)
(72) Inventor: DE BOER, Jacob, 5656 AE Eindhoven (NL)
(74) Representative: de Haan, Poul Erik

(57) **Abstract**

The present invention relates to X-ray imaging. In order to provide an X-ray imaging with improved image data for allowing an increased quality of the reconstructed 3D image, an X-ray imaging system (10) for medical imaging is provided that comprises a base structure (12), a mounting support (14), a C-arm (16) and an X-ray imaging device (18) with an X-ray source (20) and an X-ray detector (22). The X-ray source and the X-ray detector are configured to acquire a plurality of 2D X-ray images of an object for generating 3D image data of the object, and wherein the X-ray source and the X-ray detector are mounted to opposing ends of the C-arm. The C-arm is movably mounted to the base by the mounting support. Further, the mounting support is configured to provide a rotational movement (*R_{M}*) of the C-arm around a first axis (*A1*)*.* Still further, the mounting support is also configured to provide a propeller-like rotation (*R_{P}*) of the C-arm around a second axis (*A2*) in a range of at least 180°, the second axis being perpendicular to the first axis.

## Description

### FIELD OF THE INVENTION

The present invention relates to X-ray imaging with an increased angulation rotation range, and relates in particular to an X-ray imaging system for medical imaging and to a method for providing 3D image data of an object of interest.

### BACKGROUND OF THE INVENTION

For making 3D models of X-ray images, a plurality of 2D images, also referred to as slices, can be provided and then used for 3D image data generation. As an example, computed tomography is performed based on the plurality of 2D X-ray images. An example for acquiring such plurality of 2D images is a gantry with a rotating pair of source and detector. Gantries, however, are quite large and are not really compatible with interventions performed on a subject. Another example for acquiring a plurality of 2D images is a C-arm with a respective angular movement. However, it has been shown that the angular movement of the C-arm is limited and hence the image data is respectively restricted.

### SUMMARY OF THE INVENTION

There may thus be a need to need to provide an X-ray imaging with improved image data for allowing an increased quality of the reconstructed 3D image.

The object of the present invention is solved by the subject-matter of the independent claims; further embodiments are incorporated in the dependent claims. It should be noted that the following described aspects of the invention apply also for the X-ray imaging system for medical imaging and for the method for providing 3D image data of an object of interest.

According to the present invention, an X-ray imaging system for medical imaging is provided. The system comprises a base structure, a mounting support, a C-arm and an X-ray imaging device with an X-ray source and an X-ray detector. The X-ray source and the X-ray detector are configured to acquire a plurality of 2D X-ray images of an object for generating 3D image data of the object, and the X-ray source and the X-ray detector are mounted to opposing ends of the C-arm. The C-arm is movably mounted to the base by the mounting support. The mounting support is configured to provide a rotational movement of the C-arm around a first axis. The mounting support is also configured to provide a propeller-like rotation of the C-arm around a second axis in a range of at least 180°, the second axis being perpendicular to the first axis.

This provides an X-ray imaging with an increased angulation rotation range. This improves the image data available for 3D reconstruction and thus enhances the quality of 3D models based on the X-ray images

According to an example, the mounting support comprises a sleeve-like mount slidably holding the C-arm providing the rotational movement of the C-arm around the first axis. The mounting support further comprises a bearing support that rotatably connects the sleeve-like mount to the base structure. The bearing support provides the propeller-like rotation of the C-arm around the second axis in the range of at least 180°.

In an example, an X-ray imaging system for medical imaging is provided that comprises a base structure, a mounting support, a C-arm and an X-ray imaging device with an X-ray source and an X-ray detector. The X-ray source and the X-ray detector are configured to acquire a plurality of 2D X-ray images of an object for generating 3D image data of the object. The X-ray source and the X-ray detector are mounted to opposing ends of the C-arm. Further, the C-arm is movably mounted to the base by the mounting support and the mounting support comprises a sleeve-like mount slidably holding the C-arm providing the rotational movement of the C-arm around the first axis. The mounting support also comprises a bearing support that rotatably connects the sleeve-like mount to the base structure. The bearing support provides the propeller-like rotation of the C-arm around the second axis in the range of at least 180°, the second axis being perpendicular to the first axis.

According to an example, based on the propeller-like rotation by the bearing support over the range of at least 180°, the X-ray imaging device is configured to provide a first scan of X-ray images of an object in an image plane with the C-arm sliding along the sleeve-like mount, and to provide a second scan of X-ray images of the object in the image plane with the C-arm sliding along the sleeve-like mount. Further, for the first scan, the C-arm slides along a first range of orientation, and, for the second scan, the C-arm slides along a second range of orientation. The second range of orientation is at least partly opposite to the first range of orientation.

According to an example, the bearing support can be adjusted in its orientation around the second axis in a range of up to +/- 45°. The bearing support provides the propeller-like rotation around the second axis in a range of 180° around its adjusted orientation.

According to an example, an object support is provided for receiving an object of interest. The X-ray imaging system is configured to move the C-arm and the object support away from each other after a first scan such that the C-arm can rotate around the second axis and to move the C-arm and the object support again towards each other for a second scan.

According to an example, the base structure is a mobile base structure. The mobile base structure is movable along a floor surface. Further, the C-arm is movably mounted to the mobile base structure by the mounting support.

In an example, the C-arm is hold by the sleeve-like mount which is rotatably connected to the mobile base by the bearing support.

According to an example, the mobile base is provided with a wheel arrangement comprising a plurality of wheels to provide a movement along a floor surface. The mobile base comprises a front cantilevering arm extending away from the base in a main direction. The main direction refers to a direction of the C-arm covering an object on an object support. Front wheels are attached to the distal end of the front cantilevering arm. Further, the mobile base further comprises two rear cantilevering arms extending away from the base in sideways direction in relation to the main direction, wherein rear wheels are attached to each of the distal ends of the rear cantilevering arms. At least one of the rear cantilevering arms is expandable to the side to provide an increased sideward support when the C-arm is rotated around the vertical axis into the sideward position for allowing the propeller movement of e.g. 180°.

According to an example, the base structure is a floor- or ceiling-mounted fixed base structure. The C-arm is movably mounted to the fixed base structure by the mounting support. The bearing support rotatably connects the sleeve-like mount to the fixed base structure.

According to the present invention, also a method for providing 3D image data of an object of interest is provided. The method comprises the following steps:
a) acquiring a first scan of an object with an X-ray imaging system, the scan comprising a first plurality of 2D X-ray images with a C-arm along a first angular rotation of the C-arm in an image plane around a first axis;
b) rotating the C-arm around a second axis, which is perpendicular to the first axis, wherein the C-arm is rotated in a propeller-like rotation around the second axis in a range of at least 180°, the second axis being perpendicular to the first axis;
c) acquiring a second scan of the object, the scan comprising a second plurality of 2D X-ray images with the C-arm along a second angular rotation of the C-arm in the image plane around the first axis; and, as an option,
d) generating 3D image data of the object by computed tomography based on the first plurality of 2D X-ray images and the second plurality of 2D X-ray images.

According to an example, before step b), it is further provided moving the C-arm away from the object, and, before step c), it is further provided moving the C-arm back to the object into the image plane.

According to an aspect, an extension of the angulation range is provided by rotation around the propeller axis. Thus, the angular/orbital rotation can be further extended, for instance with a propeller rotation of 180°, the angulation rotation can be two times larger. In other words, the extended angulation range makes it possible to make 2D X-ray images over a range that is two times larger. As minimal angulation, a system with an angulation range of 90° plus half of the fan angle is provided. Such system will already be able to make good 3D models of X-ray images by using the propeller rotation. As an example, the bearing support of the C-arm is provided for X-ray diagnostics and treatment systems for fixed and mobile applications.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows a basic example of an X-ray imaging system in a side view.
Fig. 2A shows a side view of an example of a mobile X-ray imaging system with a C-arm movably attached by a sleeve-like mount, and Fig. 2B shows the example in a front view.
Fig. 3 shows a schematic side view of another example of a mobile X-ray with a C-arm movably attached by a two-beam support.
Fig. 4A shows an example of a mobile X-ray imaging system with a C-arm in a first propeller rotation position, and Fig. 4B shows the example with the C-arm in a second propeller rotation position.
Figs. 5A, 5B, 5C, 5D, 5E and 5F show an example with the C-arm in different angulations while the C-arm is in a first propeller rotation of 0°.
Figs. 6A, 6B, 6C, 6D, 6E and 6F show an example with the C-arm in different angulations while the C-arm is in a second propeller rotation of 180°.
Fig. 7A shows an example of a first scan in a counterclockwise direction with a range of 140°, and Fig. 7B shows a second scan in clockwise direction with a range of 140°.
Fig. 8 shows both scans combined resulting in a total scan range of 280°.
Fig. 9 partly shows a mobile base with an expandable rear cantilevering arm.
Fig. 10 shows basic steps of an example of a method for providing 3D image data of an object of interest.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

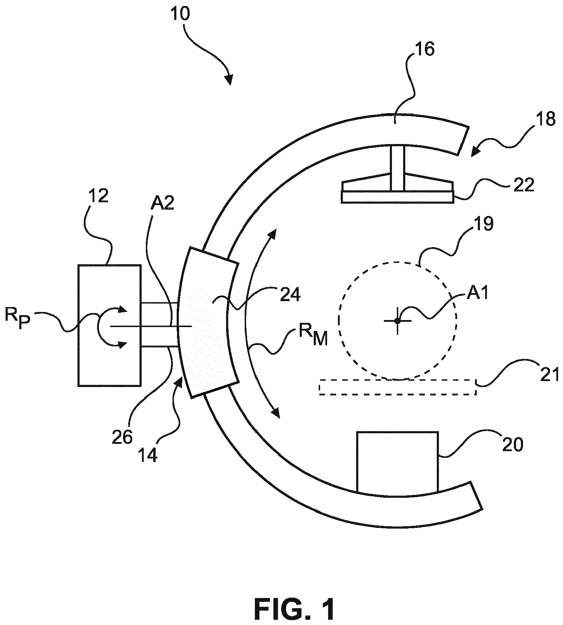

Fig. 1 schematically shows an example of an X-ray imaging system 10 for medical imaging in a side view. The system 10 comprises a base structure 12, a mounting support 14, a C-arm 16 and an X-ray imaging device 18 with an X-ray source 20 and an X-ray detector 22. The X-ray source 20 and the X-ray detector 22 are configured to acquire a plurality of 2D X-ray images of an object for generating 3D image data of the object. The X-ray source 20 and the X-ray detector 22 are mounted to opposing ends of the C-arm 16. The C-arm 16 is movably mounted to the base 12 by the mounting support 14. The mounting support 14 is configured to provide a rotational movement *R_{M}* of the C-arm 16 around a first axis *A1.* The mounting support 14 is also configured to provide a propeller-like rotation *R_{P}* of the C-arm 16 around a second axis *A2* in a range of at least 180°. The second axis *A2* is essentially perpendicular to the first axis *A1.* The term essentially perpendicular refers to exact 90°, or a range of up to +/- 5° deviating from 90°.

The term "base structure" relates to a base of the system, which provides load bearing from movable components such as the C-arm 16 to a building structure, like the floor, the walls or the ceiling of a hospital room.

The term "mounting support" relates to the construction that serves for the movable fixation of the C-arm 16 to the base 12.

The term "C-arm" refers to a structure that has two arm-like support elements for carrying the X-ray source 20 and the X-ray detector 22, while leaving a non-obstructed space between the X-ray source and the X-ray detector to be able to arrange an object in this space that is subject to the X-ray imaging. The C-arm can be provided as arc-shaped, but can also be V-shaped or U-shaped, when a mechanism is provided that allows for the angulation with the image plane. In an example, the C-arm 16 comprises a circular arm as a main part. The mounting support can thus also be referred to as C-arm suspension. The mounting support provides a dual rotational position C-arm suspension.

As an example, in an X-ray system, a so-called (constructive) image chain is provided that comprises the X-ray source 20 and the X-ray detector 22 (the latter also referred to as X-ray receptor), the C-arm 16 supporting the source and the detector, and a mount or suspension of the C-arm 16. The mount also comprises guide-ways that are used to realize the angulation/orbital rotation of the C-arm.

In an example, the X-ray source 20 and the X-ray detector 22 are defining an image centerline that starts at the focal point of the X-ray source. This image centerline is rotated with respect to the object to provide scan from different angles to achieve image data suitable for CT image data computations. The C-arm ensures that source and detector have the same distance to each other for all images. The rotation of the image centerline is done, for example, by rotating the C-arm. The rotation of the C-arm is provided around a C-arm rotation point, for example the geometric center of the circular arc of the C-arm.

This is also referred to as the Iso-centric rotation point (or Iso-center) which is located on the image chain centerline. Thus, in an example, axis *A1* is in the Iso-centric position which is located on the image chain centerline. If the C-arm centerpoint has an offset in relation to the Iso-centric rotation point, for example for mechanical balancing, a compensation is provided so the rotation will take place around the centerpoint, i.e. so the rotation will be Iso-centric.

For rotation of the C-arm, a sleeve-like support can be provided with circular bearing or movement tracks for the C-arm. By holding the sleeve-like mount in a pivoting manner around e.g. a horizontal axis, a relative arrangement of the center point and the horizontal axis is determined. The image chain can then be further extended, for example by arranging a vertical axis of the mount on the base to rotate the C-arm also to the side. This vertical axis has a determined relation to the horizontal axis. For example, they can intersect, or an offset is provided.

During imaging procedures, the constructive image chain provides rotation movement of the source detector (angulation/orbital rotation of the source detector) around the object which can be used to make 2D X-ray images. These can be used to compute 3D models/reconstructions of these 2D X-ray images. For example, multiple 2D X-ray images are generated during the image chain angulation/orbital rotation with a motorized movement, which multiple 2D images are used to generate 3D models/reconstructions.

The generation of the 3D image data is also referred to as a 3D reconstruction of a model.

The second axis *A2* is also referred to as propeller rotation axis or propeller axis. In an example, the second axis *A2* is a horizontal axis. In an alternative option, the second axis *A2* is provided in an inclined manner with respect to the horizontal plane. For example, an inclination in a range of up to +/- 5°, e.g. up to +/- 10° or up to +/- 15° is provided.

The first axis *A1* can also be provided as a horizontal axis. Alternatively, the first axis *A1* is provided in an inclined manner with respect to the horizontal plane. For example, an inclination in a range of up to +/- 5°, e.g. up to +/- 10° or up to +/- 15° is provided.

In another example, the first axis *A1* is a first horizontal axis and the second axis *A2* is a second horizontal axis.

In an option, due to angulation movement, i.e. rotation around the second axis *A2*, the first axis *A1* can have a varying orientation with respect to the horizontal plane.
In another example, the second axis *A2* has a tilted offset, i.e. the second axis *A2* is not in the horizontal plane, and the angulation rotation is also provided with that tilted offset, i.e. the first axis *A1* is also not in the horizontal plane. An example for the offset of the C-arm 16 is +/- 10° with respect to a vertical position of the C-arm 16, i.e. the first axis *A1* and the second axis *A2* have an offset of +/- 10° with respect to the horizontal plane. The term horizontal plane refers to a normal situation in a hospital, where the horizontal plane is more of less parallel to the horizontal floor plane.

For making 3D models of X-ray images, the angulation movement/orbital rotation is an attractive movement, since it can be easily executed at different positions with respect to the object lying on a table. The angulation movement is also referred to as angular movement or angulation. While the angulation/orbital rotation range may be restricted, which would limit the realization of good 3D models (3D reconstructions), the extension of the range angulation/orbital rotation range by the propeller-rotation compensates this and enables to realize better 3D models of X-ray images. Depending on the application, useful 3D models can be realized with an angulation/orbital range of 180° plus fan angle. The fan angle refers to an angular widening of the X-ray beam, which beam is fan-shaped.

The rotation of the C-arm 16 along the circular extension direction of the C-arm 16 is also referred to as angulation, orbital rotation or orbital movement. The rotation of the C-arm 16 around the horizontal axis, i.e. the second axis *A2*, is also referred to as propeller-like rotation or propeller movement.

The "rotational movement of the C-arm 16 around a first horizontal axis *A1*" can also be referred to as "angular rotation of the C-arm 16 along the circular arm defined by the C-arm 16".

The first horizontal axis *A1* is aligned with a center point of the circular arm of the C-arm 16 and the first horizontal axis *A1* runs perpendicular to a plane defined by the C-arm 16.

In an example, the second horizontal axis *A2* is also aligned with a center point of the circular arm of the C-arm 16 and the second horizontal axis *A2* runs within the plane defined by the C-arm 16 and perpendicular to the first horizontal axis *A1.*

In an example, the second horizontal axis *A2* is arranged with an offset to the center point of the circular arm of the C-arm 16, wherein a compensation for the offset is provided after the propeller rotation *R_{P}* around the second horizontal axis *A2*.

The propeller-like rotation *R_{P}* over 180° provides a duplication (multiplication by the factor of 2) of the angulation range of the C-arm 16. Hence, a C-arm 16 with an effective angulation range of up to 360° or even more degrees is provided.

In an example, image data processing is provided. The image data processing is configured to receive image data of a plurality of 2D X-ray images of an object from the X-ray imaging device 18, and to generate 3D image data of the object.

In an option, the image data processing is provided as an image data processing unit by the X-ray imaging system. The term "image data processing unit" can also be referred to as control unit. It can be provided integrated with other control functions in a central data processor or it can be provided as a separate data processor.

In another option, the image data processing is provided remote like in the cloud and the processed data is delivered back onsite to the location of the X-ray imaging system 10.

In an example, the X-ray imaging system is a fixed X-ray imaging system. In one option, the imaging device is moved away from the subject and the subject remains, wherein the base also remains. In another example, the subject is moved away from the imaging device, and the imaging device remains. In a further example, the subject and the imaging device are moved away from each other.

In another example, the X-ray imaging system is a mobile X-ray imaging system. In one option, the imaging system is moved away from the subject, and the subject remains. In another option, the subject is moved away from the imaging system, and the imaging system remains. In a further option, the subject and the imaging system are moved away from each other. It may also be provided that the imaging device is moved away from the subject and the subject remains, wherein the mobile base also remains.

In an example, the C-arm 16 is moved away from a subject, or the subject is moved away from the C-arm 16. This allows to rotate the C-arm 16 in the propeller rotation in the distanced arrangement of C-arm and subject. By this new orientation, the angulation/orbital range is extended to two times the range. With this extended range, more 2D X-ray images can be used for the 3D model/reconstruction. This will result in higher/better quality of the 3D data.

A larger angulation range also creates the possibility for smooth accelerations and decelerations, since there can be more angulation range available to realize this. This is beneficial for the dynamical behavior and has positive impact on the 3D image quality. The angulation movement can be optimized with a better dynamical behavior.

In an example, the bearing support provides the propeller-like rotation over a range of 360°. As an option, a center (or middle) imaging position is provided by the C-arm, in which the X-ray source is arranged below an object on an object support, and the X-ray source is arranged above the object on the object support. As an option, a first end imaging position is provided by the C-arm, in which the X-ray source is arranged above an object on the object support, and the X-ray source is arranged below the object on the object support. For movement from the center position to the first end position, the C-arm is moved in a first rotation direction. For example, the first rotation direction is clockwise. Further, also a second end imaging position is provided by the C-arm, in which the X-ray source is arranged above an object on the object support, and the X-ray source is arranged below the object on the object support. For movement from the center position to the second end position, the C-arm is moved in a second rotation direction, which is opposite to the first direction. For example, the first rotation direction is counter-clockwise.

In an example, a first scan is provided over a range of 280° (degrees) with the C-arm in a first rotation position covering 140° and in a second rotation position covering another 140° adding up to the resulting or effective 280°. Further, e.g. after an interventional step, an updated scan is provided along the sleeve, it may be possible to make updated 3D scans with the C-arm in a rotation position further covering 140°. This can also be the case when 2D images are made and it is made use of overlay technologies where the 3D imaging will be mapped on the 2D images. For reconstruction, the further covering scan of 140° is provided and for an adapted reconstruction procedure, the matching counterpart of the first scan is provided as priori info from the first scan to achieve complete scan data. Hence, a "sparse reconstruction" is provided using a priori info. In an example, a full scan is provided before the surgery starts, so that during surgery, only a limited scan is needed.

In Fig. 1 it is indicated as an option that the mounting support 14 comprises a sleeve-like mount 24 slidably holding the C-arm 16 providing the rotational movement of the C-arm 16 around the first axis *A1.* Further, also optionally, a bearing support 26 is provided that rotatably connects the sleeve-like mount 24 to the base structure 12. The bearing support 26 provides the propeller-like rotation of the C-arm 16 around the second axis *A2* in the range of at least 180°. However, it is noted that the sleeve-like mount 24 and the bearing support 26 are provided as an option where the C-arm 16 is arc-shaped. Also, other examples for the mounting support 14 are provided like an integrated joint allowing the required movements. As another option, an object support 21 is provided for receiving an object 19 of interest. The object support 21 may be provided as a patient table. The X-ray imaging system 10 is configured to move the C-arm 16 and the object support 21 away from each other such that the C-arm 15 can rotate around the second axis *A2*.

The "object" may also be referred to as subject or individual. The subject may further also be referred to as patient, although it is noted that this term does not indicate whether any illness or disease is actually present with the subject.

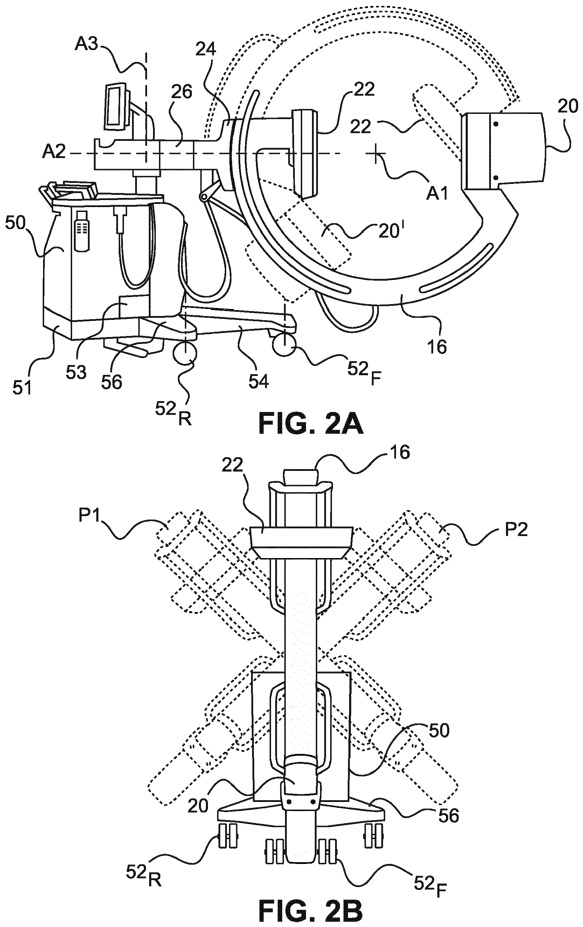

Figs. 2A and 2B show an example of the X-ray imaging system 10 where the base structure is provided as a mobile base structure 50. Fig. 2A shows a side view of the example and Fig. 2B shows the example in a front view. The mobile base structure 50 is movable along a floor surface, for example by wheels 52 or rollers. The C-arm 16 is movably mounted to the mobile base structure by the mounting support 14. As an option, the C-arm is hold by the sleeve-like mount 24 that is rotatably connected to the mobile base 50 by the bearing support 26.

The sliding of the C-arm 16 along the sleeve-like mount 24 allows different positions for X-ray imaging. In Fig. 2A, as an example, a second position (out of many possible) is indicated with hashed lines for the X-ray source with reference numeral 20' and for the X-ray detector with reference numeral 22'.

In an example, the mobile base 50 is provided with a wheel arrangement comprising a plurality of wheels 52 to provide a movement along the floor surface. For example, the mobile base 50 comprises a front cantilevering arm 54 extending away from the base in a main direction, wherein front wheels 52_{F} are attached to a distal end of the front cantilevering arm 54. In an option, two front cantilevering arms 54 are provided. The mobile base 50 further comprises two rear cantilevering arms 56 extending away from the base in sideways direction in relation to the main direction, wherein rear wheels 52_{R} are attached to each of the distal ends of the rear cantilevering arms 56.

For example, an imaging system, also referred to as surgery imaging system or surgery system, has an angulation movement/orbital range of 140°. This range is doubled after the 180° propeller rotation. This means that the complete range will be e.g. 2 x 140° = 280°. In an example, an angulation movement of 180° plus fan angle is good enough to make good 3D models/reconstructions, such that the angular/orbital range of the C-arm can be reduced which results in a reduced construction of the sleeve-like mount.

For example, the C-arm 16 (also referred to as C-arc) has a circular extension direction and the sleeve-like mount holds the C-arm slidable along the circular extension direction. The C-arm 16 is slidably hold by the sleeve-like mount 24, which itself is rotatable around a horizontal axis of rotation, i.e. around the second axis *A2*.

The effective, or resulting, angulation range of the X-ray imaging system is twice the angulation range provided by the sleeve-like mount itself. The bearing support provides a dual position angulation, that can also be referred to as opposite position angulation.

As an option, Fig. 2B indicates that the bearing support 26 can be adjusted in its orientation around the second axis in a range of up to +/- 45°. A first adjusted position *P1* is indicated with hashed lines as a counterclockwise adjustment of approximately 45°. A second adjusted position *P2* is indicated with hashed lines as a clockwise adjustment of approximately 45°.

However, the bearing support 26 still provides the propeller-like rotation around the second axis in the range of 180° around its respectively adjusted orientation, i.e. 180° in relation to the adjusted position, like the first or second adjusted positions *P1*, *P2.*

The bearing support 26 thus comprises a two-way rotation. The propeller rotation over 180° provides the arrangement of two scans in the same image plane but with two opposing viewing directions. The adjustment rotation over +/- 45° (relating to the vertical orientation) provides the possibility of an adjusted (e.g. vertical or inclined) orientation of the image plane.

In an option, the two-way rotation is provided within one rotation by providing an extended angulation range.

As an option, the bearing support 26 is movably mounted to the base structure, e.g. the mobile base 50, to be rotatable around a vertical axis, also referred to as a third axis *A3.* For rotation of the C-arm 16 around the second axis *A2,* the C-arm 16 can be rotated to the side around the vertical axis, i.e. the third axis *A3.* The C-arm 16 can thus be swung to the side of the patient support and the cantilevered wheels provide sufficient support to prevent a tipping over when the weight arrangement of the C-arm 16 is changed.

As another option, the mobile base 50 comprises a driving mechanism 51 to activate movement of the base away from an object and towards the object, and a positioning device 53 for repositioning the C-arm 16 after removing it for rotation around the second axis *A2.*

In an example, the mobile base structure 50 comprises a frame structure for transferring the load of the C-arm 16 from the mounting support to a ground surface. In an example, the mobile base structure comprises a housing enclosing the frame structure.

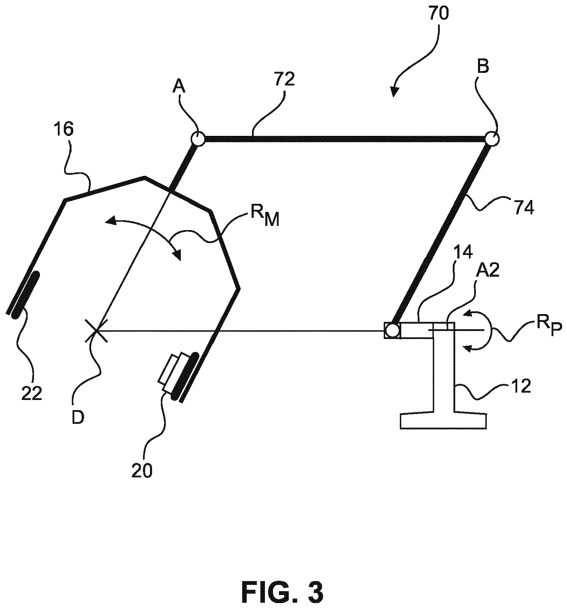

Fig. 3 shows a schematic side view of another example of the mobile X-ray with a C-arm 16 movably attached by a two-beam support structure 70. The support structure 70 comprises a primary supporting beam 72 and a secondary supporting beam 74. An example of the base 12 is also provided. The C-arm 16 holds the X-ray detector 22 at a first end, and the X-ray source 20 at a second end, with a connection line (not shown) in between, on which an Iso-center D can be located. The C-arm 16 is connected to the primary supporting beam 72 in a first pivotable connection point A with a first connector, and the primary supporting beam 72 is connected to the secondary supporting beam 74 in a second pivotable connection point B with a second connector, and the secondary supporting beam 74 is connected to the base 12 in a third pivotable connection point C with a third connector. Further, for allowing the rotational movement in a range of at least 180°around the second axis A2, i.e. the propeller-like rotation RP, the mounting support 14 is arranged between the third pivotable connection point C and the base 12. The first, second and third pivotable connection points A, B, C and the Iso-center D define a parallelogram. An angulation rotation movement of the support structure around the Iso-center D is realized such that a parallelogram (A-B-C-D) movement is realized. In an example, the C-arm 16, the primary supporting beam 72, the secondary supporting beam 74, the base 12 and a connection line between the base and the Iso-center D are coupled in a way that the connection lines, i.e. the first, second, and third pivotable connection points A, B, C and the Iso-center D remain in a parallelogram shape during a movement of the imaging support arrangement around the Iso-center D in an Iso-centered angulation rotation, i.e. the rotational movement RM. The Iso-center remains in its initial position.

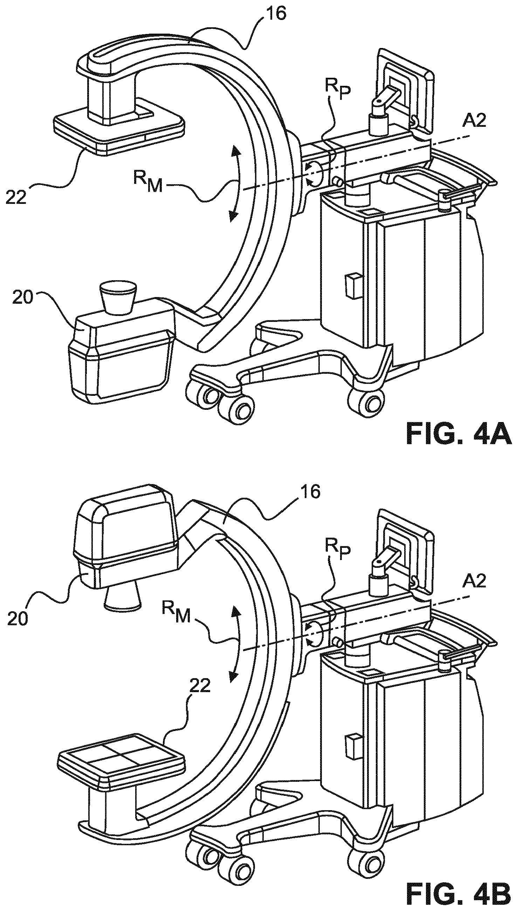

Fig. 4A shows the mobile X-ray imaging system with the C-arm 16 in a first propeller rotation position *PRP1*, and Fig. 4B shows the example with the C-arm in a second first propeller rotation position *PRP2.* In each of the propeller positions, the rotational movement *R_{M}* of the C-arm 16 is provided.

In an option, based on the propeller-like rotation by the bearing support over the range of at least 180°, the X-ray imaging device is configured to provide a first scan of X-ray images of an object in an image plane with the C-arm sliding along the sleeve-like mount, and to provide a second scan of X-ray images of the object in the image plane with the C-arm sliding along the sleeve-like mount. For the first scan, the C-arm slides along a first range of orientation, and, for the second scan, the C-arm slides along a second range of orientation. The second range of orientation is at least partly opposite to the first range of orientation.

The image plane defines a viewing plane for the X-ray imaging. When the X-ray radiation is provided by a fan-shaped beam, the viewing plane is provided as a viewing slice or viewing layer, i.e. a plane with a certain "thickness".

In an example, the sleeve-like mount provides a rotation angle of 90° around the first horizontal axis. In the first vertical orientation, the C-arm rotates around the object in a first range, and in the second vertical orientation, the C-arm rotates around the object in a second range. The first range and the second range are arranged such that a total range of 180° is provided.

In another example, the sleeve-like mount provides a rotation angle of 180° around the first horizontal axis. In the first vertical orientation, the C-arm rotates around the object in the first range, and in the second vertical orientation, the C-arm rotates around the object in the second range. The first range and the second range are arranged such that a total range of 360° is provided.

In an example, not shown in detail, the bearing support comprises a bearing joint with a first and a second stop position limiting the range of rotation around the second axis to 180°. In an option, the bearing stops can be adjusted to provide an image plane with a vertical orientation or an image plane with an inclined orientation.

In another example, the stop positions are programmed, and the accuracy of a drivetrain realizes the needed reproduceable accuracy at required positions.

The terms vertical and inclined relate to the horizontal floor plane of a normal hospital room. For example, the term inclined relates to a range of up to +/- 45° from the vertical direction.

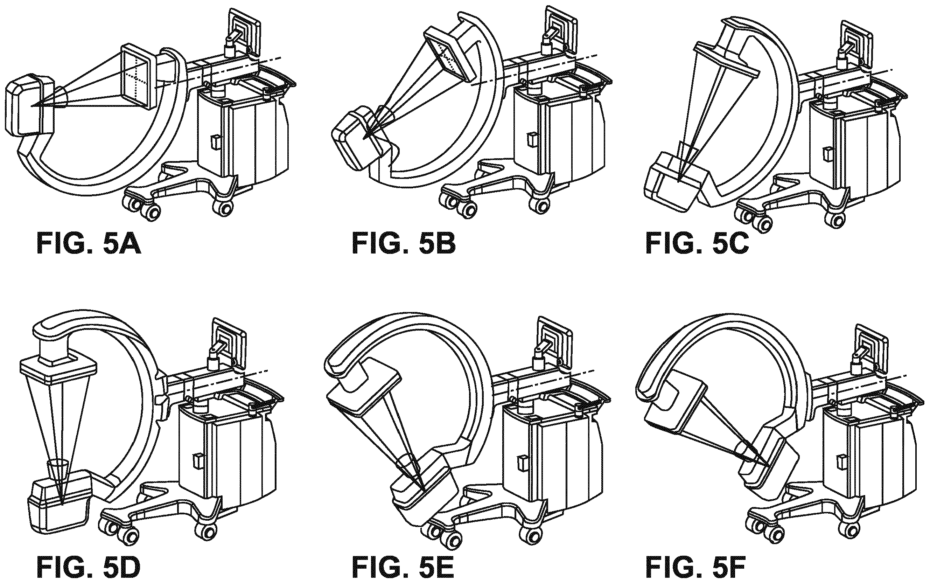

Figs. 5A, 5B, 5C, 5D, 5E and 5F show an example with the C-arm in different angulations while the C-arm is in a first propeller rotation of e.g. 0°, for example in the first propeller rotation position *PRP1.* Fig. 5A shows an angulation at 0°, Fig. 5B an angulation at 30°, Fig. 5C an angulation at 60°, Fig. 5D an angulation at 90°, Fig. 5E an angulation at 120° and Fig. 5F an angulation at 140°.

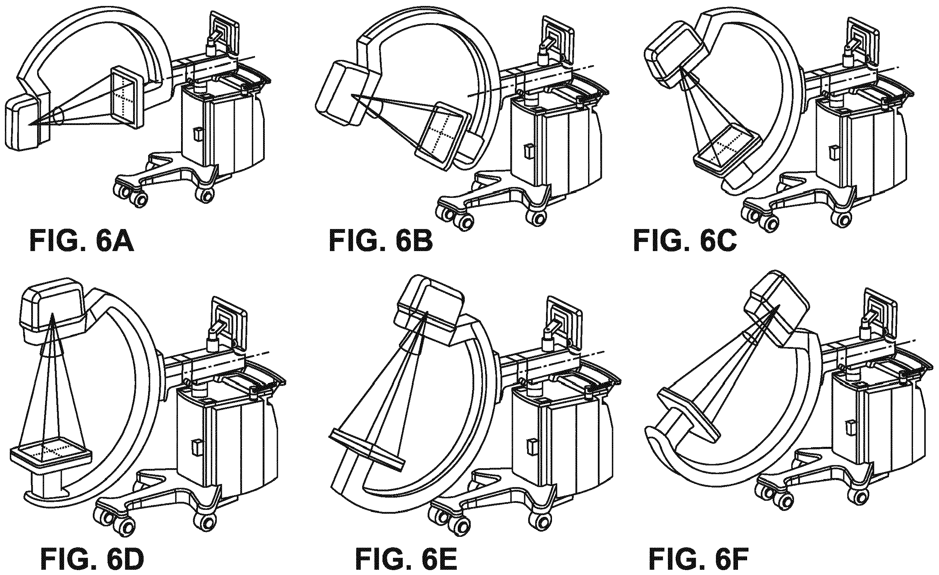

Figs. 6A, 6B, 6C, 6D, 6E and 6F show an example with the C-arm in different angulations while the C-arm is in a second propeller rotation of e.g. 180°, for example in the second propeller rotation position *PRP2.* Fig. 6A shows an angulation at 0°, Fig. 6B an angulation at 30°, Fig. 6C an angulation at 60°, Fig. 6D an angulation at 90°, Fig. 6E an angulation at 120° and Fig. 6F an angulation at 140°.

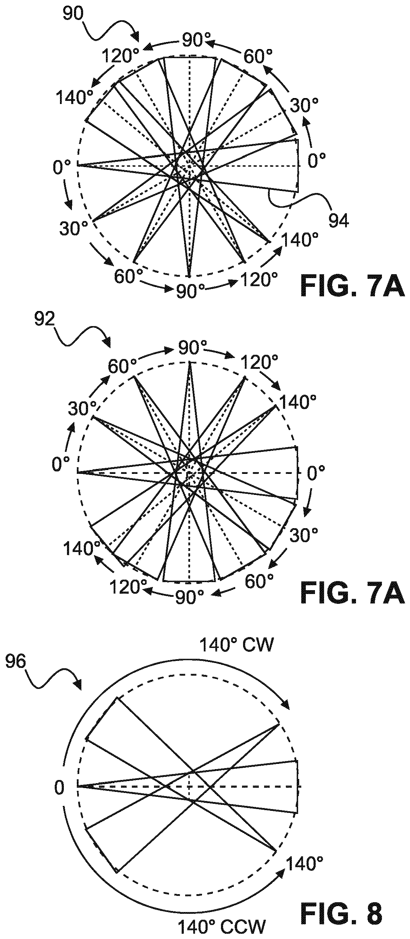

Fig. 7A shows an example where the sleeve-like mount provides a rotation angle of 140° around the first horizontal axis. In the first vertical orientation, the C-arm rotates around the object in a first range, for example providing a first scan 90 in a counterclockwise (*CCW*) direction with a range of 140°. In the second vertical orientation, after the propeller rotation, the C-arm rotates around the object in a second range, for example providing a second scan 92 in a clockwise (*CW*) direction with a range of 140°. For better visibility, only a few exemplary X-ray beams 94 are indicated, for example provided in steps of 30°.

Fig. 8 shows that the first range and the second range are arranged such that a scan 96 with a total range of 280° is provided. The combined scan is indicated where both scans *CCW*+*CW* are used to make a high-quality 3D model of the scanned X-ray images. In this exemplary case, both scan ranges are 140°; and with a 180° propeller rotation, the complete combined scan range is 280°.

In a further example, not shown in detail, the bearing support provides the propeller-like rotation around the second axis in a range of 270° plus fan angle of an X-ray beam provided by the X-ray source.

In another example, not shown in detail, the base structure is a floor- or ceiling-mounted fixed base structure. The C-arm is movably mounted to the fixed base structure by the mounting support, and the bearing support rotatably connects the sleeve-like mount to the fixed base structure.

In an option, the bearing support comprises a vertical arm for suspending the C-arm from a ceiling. In another option, the bearing support comprises an upright arm for supporting the C-arm from a floor region.

As an option, the bearing support comprises an upper (when ceiling mounted) or lower (when floor mounted) rotating joint connection, allowing a swiveling around a vertical axis.

In a further option, the bearing support comprises a horizontal arm for mounting the C-arm to a wall structure. As an option, the bearing support comprises a rotating wall joint connection allowing a swiveling around a vertical axis.

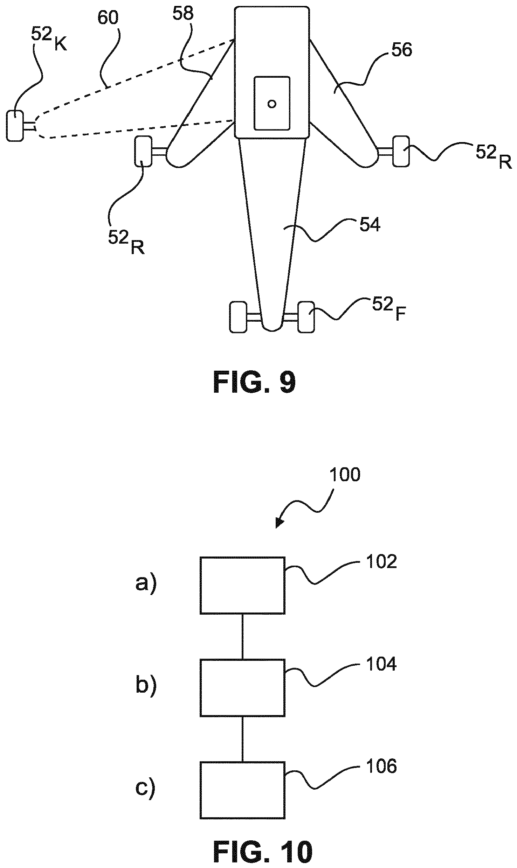

Fig. 9 partly shows an example of the mobile base 50. As an option, one 58 of the rear cantilevering arms 56 is expandable to the side (here to the left side) to provide an increased sideward support when the C-arm 16 is rotated around the vertical axis. The expandable rear cantilevering arm is indicated with reference numeral 58, and the expanded state is sown in hashed lines 60.

Fig. 10 shows an example of a method 100 for providing 3D image data of an object of interest. The method 100 comprises the following steps:
- In a first step 102, also referred to as step a), a first scan of an object is acquired with an X-ray imaging system. The first scan comprises a first plurality of 2D X-ray images with a C-arm along a first angular rotation of the C-arm in an image plane around a first axis.
- In a second step 104, also referred to as step b), the C-arm is rotated around a second axis, which is perpendicular to the first axis, wherein the C-arm is rotated in a propeller-like rotation around the second axis in a range of at least 180°, the second axis being perpendicular to the first axis.
- In a third step 106, also referred to as step c), a second scan of the object is acquired. The second comprises a second plurality of 2D X-ray images with the C-arm along a second angular rotation of the C-arm in the image plane around the first axis.
- In a fourth step 108, also referred to as step d), provided as an option, 3D image data of the object is generated by computed tomography based on the first plurality of 2D X-ray images and the second plurality of 2D X-ray images.

In an example, before step b), it is further provided moving the C-arm away from the object; and, before step c), it is further provided moving the C-arm back to the object into the image plane.

In an example, the X-ray imaging system comprises a mobile base structure; and it is provided to move the X-ray imaging system away from the object for step b) and to reposition the X-ray imaging system for step c).

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An X-ray imaging system (10) for medical imaging, comprising:
- a base structure (12);
- a mounting support (14);
- a C-arm (16); and
- an X-ray imaging device (18) with an X-ray source (20) and an X-ray detector (22);
wherein the X-ray source and the X-ray detector are configured to acquire a plurality of 2D X-ray images of an object for generating 3D image data of the object, and wherein the X-ray source and the X-ray detector are mounted to opposing ends of the C-arm;
wherein the C-arm is movably mounted to the base by the mounting support;
wherein the mounting support is configured to provide a rotational movement (RM) of the C-arm around a first axis (A1);
wherein the mounting support is also configured to provide a propeller-like rotation (RP) of the C-arm around a second axis (A2) in a range of at least 180°, the second axis being perpendicular to the first axis.

2. System according to claim 1, wherein the mounting support comprises:
- a sleeve-like mount (24) slidably holding the C-arm providing the rotational movement of the C-arm around the first axis; and
- a bearing support (26) that rotatably connects the sleeve-like mount to the base structure; and
wherein the bearing support provides the propeller-like rotation of the C-arm around the second axis in the range of at least 180°.

3. System according to claim 2, wherein, based on the propeller-like rotation by the bearing support over the range of at least 180°, the X-ray imaging device is configured to provide a first scan of X-ray images of an object in an image plane with the C-arm sliding along the sleeve-like mount, and to provide a second scan of X-ray images of the object in the image plane with the C-arm sliding along the sleeve-like mount; and
wherein, for the first scan, the C-arm slides along a first range of orientation, and wherein, for the second scan, the C-arm slides along a second range of orientation; the second range of orientation being at least partly opposite to the first range of orientation.

4. System according to claim 1, 2 or 3, wherein the second axis is a horizontal axis.

5. System according to one of the preceding claims, wherein the bearing support provides the propeller-like rotation around the second axis in a range of 270° plus fan angle of an X-ray beam provided by the X-ray source.

6. System according to one of the preceding claims, wherein the bearing support can be adjusted in its orientation around the second axis in a range of up to +/- 45°; and
wherein the bearing support provides the propeller-like rotation around the second axis in a range of 180° around its adjusted orientation.

7. System according to one of the preceding claims, wherein an object support is provided for receiving an object of interest; and
wherein the X-ray imaging system is configured to move the C-arm and the object support away from each other such that the C-arm can rotate around the second axis.

8. System according to one of the preceding claims, wherein the bearing support is movably mounted to the base structure to be rotatable around a vertical axis; and
wherein, for rotation of the C-arm around the second axis, the C-arm can be rotated to the side around the vertical axis.

9. System according to one of the preceding claims, wherein the base structure is a mobile base structure (50);
wherein the mobile base structure is movable along a floor surface;
wherein the C-arm is movably mounted to the mobile base structure by the mounting support.

10. System according to claim 9, wherein the mobile base is provided with a wheel arrangement comprising a plurality of wheels (52) to provide a movement along a floor surface;
wherein the mobile base comprises a front cantilevering arm extending away from the base in a main direction, wherein front wheels are attached to the distal end of the front cantilevering arm;
wherein the mobile base further comprises two rear cantilevering arms extending away from the base in sideways direction in relation to the main direction, wherein rear wheels are attached to each of the distal ends of the rear cantilevering arms;
wherein one (58) of the rear cantilevering arms is expandable to the side to provide an increased sideward support when the C-arm is rotated around the vertical axis.

11. System according to one of the preceding claims, wherein the mobile base comprises a driving mechanism (51) to activate movement of the base away from an object and towards the object, and a positioning device for repositioning the C-arm after removing it for rotation around the second axis.

12. System according to one of the preceding claims, wherein the base structure is a floor- or ceiling-mounted fixed base structure;
wherein the C-arm is movably mounted to the fixed base structure by the mounting support; and
wherein the bearing support rotatably connects the sleeve-like mount to the fixed base structure.

13. A method (100) for providing 3D image data of an object of interest, comprising the following steps:
a) acquiring (102) a first scan of an object with an X-ray imaging system, the scan comprising a first plurality of 2D X-ray images with a C-arm along a first angular rotation of the C-arm in an image plane around a first axis;
b) rotating (104) the C-arm around a second axis, which is perpendicular to the first axis, wherein the C-arm is rotated in a propeller-like rotation around the second axis in a range of at least 180°, the second axis being perpendicular to the first axis;
c) acquiring (106) a second scan of the object, the scan comprising a second plurality of 2D X-ray images with the C-arm along a second angular rotation of the C-arm in the image plane around the first axis; and
d) generating (108) 3D image data of the object by computed tomography based on the first plurality of 2D X-ray images and the second plurality of 2D X-ray images.

14. A computer program element for controlling an apparatus according to one of the claims 1 to 12, which, when being executed by a processing unit, is adapted to perform the method steps of claim 13.

15. A computer readable medium having stored the program element of claim 14.
